# EUROPEAN PATENT APPLICATION

(11) **EP 2 014 308 A2**
(43) Date of publication of application: **14.01.2009**
(21) Application number: 08252335.8
(22) Date of filing: 09.07.2008
(51) Int. Cl.: A61K 47/48, A61L 31/10, A61L 31/16, C08L 67/04

(54) **A coating employing an anti-thrombotic conjugate**

(30) Priority: 10.07.2007 US 775256
(71) Applicant: Cordis Corporation, Miami Lakes, FL 33014 (US)
(72) Inventor: Zhao, Jonathan, Belle Mead NJ 08502 (US)
(74) Representative: Belcher, Simon James

(57) **Abstract**

A biodegradable antithrombotic conjugate having heparin and other anti-thrombotic moieties are introduced as side chains to the polymer backbone modified by a click chemistry reaction. Various bioabsorbable monomers and dimers such as valerolactone may be used in the monomer derivation, homo- and co-polymerization, and the conjugation with a biologically active molecule by click chemistry. A coating comprising a biocompatible and bioabsorbable polymer anti-thrombotic conjugate is applied to at least a portion of an implantable device to prevent or reduce the formation of thrombosis on the surface of the implantable device. A first or sub-layer of the coating is prepared by mixing a polymeric material and a biologically active agent with a solvent, thereby forming a homogeneous solution. A second or outer layer comprising the present anti-thrombotic conjugate may be applied over the inner drug-containing layers using, for example, a dip coating or spray coating process.

## Description

The present invention relates to a material for application to at least a portion of the surface of an article or for implantation within an article. In particular, this invention relates to a bioabsorbable polymer having an anti-thrombotic composition conjugated therewith wherein an anti-restenotic agent may be contained within the polymer matrix of the bioabsorbable polymer. This invention also relates to a device having the conjugate coated to its surface or contained within the device itself.

Stenosis is the narrowing or constriction of a vessel resulting from the buildup of fat, cholesterol, and other substances over time. In severe cases, stenosis can completely occlude a vessel. Interventional procedures have been employed to open stenosed vessels. One example of an interventional procedure is percutaneous transluminal coronary angioplasty (PTCA) or balloon coronary angioplasty. In this procedure, a balloon catheter is inserted and expanded in the constricted portion of the vessel for clearing a blockage. About one-third of patients who undergo PTCA suffer from restenosis, wherein the vessel becomes blocked again, within about six months of the procedure. Thus, restenosed arteries may have to undergo another angioplasty.

Restenosis can be inhibited by a common procedure that consists of inserting a stent into the effected region of the artery instead of, or along with, angioplasty. A stent is a tube made of metal or plastic, which can have either solid walls or mesh walls. Most stents in use are metallic and are either self-expanding or balloon-expandable. The decision to undergo a stent insertion procedure depends on certain features of the arterial stenosis. These include the size of the artery and the location of the stenosis. The function of the stent is to buttress the artery that has recently been widened using angioplasty, or, if no angioplasty was used, the stent is used to prevent elastic recoil of the artery. Stents are typically implanted via a catheter. In the case of a balloon-expandable stent, the stent is collapsed to a small diameter and slid over a balloon catheter. The catheter is then maneuvered through the patient's vasculature to the site of the lesion or the area that was recently widened. Once in position, the stent is expanded and locked in place. The stent stays in the artery permanently, holds it open, improves blood flow through the artery, and relieves symptoms (usually chest pain).

Stents are not completely effective in preventing restenosis at the implant site. Restenosis can occur over the length of the stent and/or past the ends of the stent. Physicians have recently employed new types of stents that are coated with a thin polymer film loaded with a drug that inhibits smooth cell proliferation. The coating is applied to the stent prior to insertion into the artery using methods well known in the art, such as a solvent evaporation technique. The solvent evaporation technique entails mixing the polymer and drug in a solvent. The solution comprising polymer, drug, and solvent can then be applied to the surface of the stent by either dipping or spraying. The stent is then subjected to a drying process, during which the solvent is evaporated, and the polymeric material, with the drug dispersed therein, forms a thin film layer on the stent.

The release mechanism of the drug from the polymeric materials depends on the nature of the polymeric material and the drug to be incorporated. The drug diffuses through the polymer to the polymer-fluid interface and then into the fluid. Release can also occur through degradation of the polymeric material. The degradation of the polymeric material may occur through hydrolysis or an enzymatic digestion process, leading to the release of the incorporated drug into the surrounding tissue.

An important consideration in using coated stents is the release rate of the drug from the coating. It is desirable that an effective therapeutic amount of the drug be released from the stent for a reasonably long period of time to cover the duration of the biological processes following an angioplasty procedure or the implantation of a stent. Burst release, a high release rate immediately following implantation, is undesirable and a persistent problem. While typically not harmful to the patient, a burst release "wastes" the limited supply of the drug by releasing several times the effective amount required and shortens the duration of the release period. Several techniques have been developed in an attempt to reduce burst release. For example, US-6258121 discloses a method of altering the release rate by blending two polymers with differing release rates and incorporating them into a single layer.

A potential drawback associated with the implantation of a drug eluting stent (DES) is that thrombosis may occur at different times following implantation or deployment. Thrombosis is the formation of blood clots on or near an implanted device in the blood vessel. The clot is usually formed by an aggregation of blood factors, primarily platelets and fibrin, with entrapment of cellular elements. Thrombosis, like stenosis, frequently causes vascular obstruction at the point of its formation. Both restenosis and thrombosis are two serious and potentially fatal conditions that require medical intervention. But, treating one condition may lead to the presence of the other condition. A thrombus formation on the surface of a stent is frequently lethal, leading to a high mortality rate of between 20 to 40% in the patients suffering from a thrombosis in a vessel.

One way to address the formation of stent thrombosis is through the use of an anticoagulant such as a heparin. Heparin is a substance that is well known for its anticoagulation ability. It is known in the art to apply a thin polymer coating loaded with heparin onto the surface of a stent using the solvent evaporation technique. For example, US-5837313 discloses a method of preparing a heparin coating composition. A drawback to the use of heparin, however is that it does not co-exist well with agents that prevent restenosis. For example, if heparin is mixed with an anti-thrombotic agent within a polymer coating, the hydrophilic nature of heparin will interfere with the desired elution profile for the anti-restenotic agent. For example, therapeutic agent is embedded in the matrix of a polymer coating by solvent processing. If an anti-coagulant is also embedded in the polymer matrix, it will attract water in an uncontrolled manner. This can happen during manufacturing or when the coated device is implanted and will adversely affect the stability or efficacy of the agent and/or interfere with the desired elution profile.

Nonetheless, several approaches have been proposed for combining anti-thrombotic and therapeutic agents within the coatings for an implantable medical device. US-5525348 discloses a method of complexing pharmaceutical agents (including heparin) with quarternary ammonium components or other ionic surfactants and bound with water insoluble polymers as an antithrombotic coating composition. This method suffers from the possibility of introducing naturally derived polymer such as cellulose, or a derivative thereof, which is heterogeneous in nature and may cause unwanted inflammatory reactions at the implantation site. These ionic complexes between an antithrombotic agent such as heparin and an oppositely charged carrier polymer may also negatively affect the coating integration, and if additional pharmaceutical agents are present, may affect the shelf stability and release kinetics of these pharmaceutical agents.

A slightly different approach is disclosed in US-6702850, US-6245753 and US-7129224 in which antithrombotic agents, such as heparin, are covalently conjugated to a non-absorbable polymer, such as a polyarylic acid, before use in a coating formulation. The overall hydrophobicity of these conjugates is further adjusted by addition of a hydrophobic agent such as octadecylamine, which is an amine with a long hydrocarbon chain. This approach has several potential disadvantages such as the known toxicity of polyacrylic acid after heparin is metabolized in vivo. The addition of a hydrophobic amine also raises the concern of tissue compatibility and reproduction of the substitution reactions of each step. Moreover, the remaining components of the coating are not biodegradable.

Another antithrombotic coating approach is disclosed in US-6559132, US-6461665 and US-6767405 in which a carrier molecule such as chitosan is conjugated to an activated metal surface of a medical device. Thereafter, heparin is covalently conjugated to an intermediate molecule. This process may be repeated several times until a desired antithrombotic layer is achieved. Alternatively, this coating can be achieved in a batch process mode. This approach, however, is not readily applicable to a medical device that is coated with a polymer coating that contains pharmaceutical agent/s. Some of these successful anti-restenotic agents such as sirolimus may be damaged during these conjugating processes, especially these processes where aqueous processes are involved.

WO-2005/097223 discloses a method wherein a mixture of heparin conjugated with photoactive crosslinkers with dissolved or dispersed with other durable polymers such as Poly(butyl methacrylate) and poly(vinyl pyrrolidone) in a same coating solution and crosslinked with UV light in the solution or after the coating is applied. The potential disadvantage of this approach is that the incorporated drug/s may be adversely affected by the high energy UV light during crosslinking process, or worse, the drug/s may be crosslinked to the matrix polymers if they possess functional groups that may be activated by the UV energy.

US-A-2005/0191333, US-A- 2006/0204533 and WO-2006/099514 disclose the use of a low molecular weight complex of heparin and a counter ion (stearylkonium heparin), or a high molecular weight polyelectrolyte complex, such as dextran, pectin to form a complex form of an antithrombotic entity. These antithrombotic complexes are further dispersed in a polymer matrix that may further comprise a drug. Such approaches create a heterogeneous matrix of a drug and a hydrophilic species of heparin wherein the hydrophilic species attract water before and after the implantation to adversely affect the stability and release kinetics of the drug. In addition, the desired antithrombotic functions of heparin and similar agent should be preferably located on the surface, not being eluted away from the surface of a coated medical device.

There remains a need for a coating material that can satisfy the stringent requirements, as described above, for applying on at least one surface of a medical device and can be prepared through a process that is compatible with the sensitive pharmaceutical or therapeutic agents impregnated in the coatings. This helps to fill a need for a coating that treats both restenosis and prevents thrombosis when applied to the outer surface of a drug eluting stent.

According to the present invention a biologically active molecule, such as heparin, is grafted to a biocompatible and bioabsorbable polyester via a click chemistry process. In particular, a click chemistry process is employed to introduce functionality such as anti-thrombotic properties via a heparin molecule to the side chains of block polyesters taking advantage of pendant unsaturation and involving fewer steps in transformation and greater versatility. Typically, a click chemistry process utilizes a reaction between a terminal alkyne and an azide to form a triazole in the presence of a catalyzing agent. For example, the method(s) described herein may employ a stepwise analogue of the Cu(I)-catalyzed Huisgen 1,3-dipolar cycloaddition of azides and alkynes to form a triazole linkage.

Click chemistry results in triazole formation and provides a method for coupling a wide-range of molecules in a regiospecific fashion under relatively mild reaction conditions with few byproducts. Click chemistry provides the easy introduction of azide and alkyne groups into organic and polymer molecules, stability of these groups to many reaction conditions, and the tolerance of the reaction to the presence of other functional groups. Thus, the application of click chemistry to aliphatic polyesters is ideal given the sensitivity of the polyester backbone to the conditions required for many conventional organic transformations and coupling. For example, a click reaction of aliphatic polyesters, bearing pendant acetylenes with azide-terminated biological molecules, such as a heparin molecule may be employed with the present invention.

Also disclosed are coatings and similar processes wherein polyester heparin conjugates of the present invention are applied to at least a portion of an implantable medical device. This invention has a broad spectrum of applications since it applies to almost all known polyesters made via a ring opening polymerization process. The alkyne functional group may be introduced to the alpha position to the carbonyl group of a cyclic lactone. These modified lactone monomers can then polymerize to yield a homopolymer polyester with pendant alkyne side groups. Polyesters are generally limited in scope due to their hydrophobic and semi-crystalline properties and the absence of functionality along the polymer backbone, which could otherwise be used for modifying physical and chemical properties and introducing bioactive moieties. Modifications to the polymer chain via click chemistry will impart specific biological functions of the functional moieties to the polyester copolymers. In addition, the density of the alkyne side groups may be adjusted by varying the ratio of the modified lactone monomer and the unmodified lactone monomer. The modified lactone monomer may also be copolymerized with a different lactone monomer or dimer such as lactide, glycolide etc. to adjust the physical and chemistry properties of the copolymers.

Pendant functionality of aliphatic polyesters can be achieved by polymerization of functionalized lactones, post-polymerization modifications, or a combination of these two approaches. When polymerizing functionalized lactones, the functionality must be compatible with the polymerization conditions and any subsequent chemical processes. The functional group should not interfere with the ring opening polymerization, and should also be robust enough to survive all these processes.

A conjugate between a heparin and a bioabsorbable polymer and a device having the conjugate applied to its surface or embedded within its structure is also provided. The outmost layer of the coating comprises the conjugate of the present invention, which prevents the formation of thrombosis, and also serves to modulate the release kinetics of the agent(s) contained within an inner layer(s) of the coating.

A first or sub-layer of the coating is prepared by mixing a polymeric material and a biologically active agent with a solvent, thereby forming a homogeneous solution. The polymeric material can be selected from a wide range of synthetic materials, but in one exemplary embodiment, a poly(lactide-to-glycolide) (PLGA) is used. The biologically active agent is selected depending on the desired therapeutic results. For example, an antiproliferative drug such as paclitaxel, an immunosuppressant, such as a rapamycin, and/or anti-inflammatory drug, such as dexamethasone, may be included in the inner layer. Once prepared, the solution can be applied to the device through a dipping or spraying process. During drying, the solvent evaporates, and a thin layer of the polymeric material loaded with the biologically active agent is left coated over the stent. One or more distinct biologically active agents can be added via providing additional layers with a biologically active agent.

The second or outer layer comprises an anti-thrombotic heparin-bioabsorbable polymer conjugate. This coating may be applied over the inner drug-containing layers using, for example, a dip coating or spray coating process. In one exemplary embodiment of the present invention, the outer layer comprising an anti-thrombotic heparin-bioabsorbable polymer conjugate that may be dissolved in a mixed solvent system comprising ethyl acetate (EA) and isopropanol (IPA). The solution is then sprayed onto the surface of the device that has already been coated with the agent-containing layer as described above. After drying, the anti-thrombotic heparin bioabsorbable polymer conjugate remains in the outer layer of the coating, allowing agent from the inner layer to be eluted there through.

The coated device may be implanted into an afflicted area of a body, for example, a vessel like the coronary artery, using an appropriate procedure that depends on the properties of the device. The device may comprise a scaffold that will hold the vessel open, for example, a stent. The biologically active agent will be released from the first layer, thereby providing the desired therapeutic result, such as inhibiting smooth cell proliferation. The anti-thrombotic heparin-bioabsorbable polymer conjugate in the outmost layer becomes partially hydrated and prevents blood coagulation on and around the device, thus inhibiting thrombosis and sub-acute device thrombosis. In addition, the anti-thrombotic heparin-bioabsorbable polymer conjugate in the outmost layer may additionally reduce or prevent the burst release of the biologically active agent from the inner drug containing layer, thereby allowing the release to occur over a relatively extended period of time.

Another alternative is to form a particle utilizing the polymer and heparin conjugate as a carrier for a therapeutic agent within its polymer matrix. In this embodiment the agent is somewhat associated with the hydrophobic core of the polymer. The agent is co-dissolved with the conjugate using a solvent that is later evaporated creating particles with the agent at their core. These particles are ideally suited for placement within the structure of a device. For example, a device may have structural features such as wells, indentations, folds, or channels having particles therein. This allows for particles having differing properties to be placed at various locations along the device. Moreover, particles having at least two different agents can be located within the same structural feature. Agent is released from the structural feature as the particles degrade. Simultaneously, the presence of heparin will prevent thrombosis at the placement site of the device.

Embodiments of the invention are described below by way of example with reference to the accompanying drawings, in which:
Figure 1 is a schematic representation of introduction of an acetylene functional group to a lactone monomer and its subsequent copolymerization with a caprolactone comonomer to form a functionalized polyester copolymer via a ring opening polymerization with ethanol as the initiator.
Figure 2 is a reaction scheme introducing an azide end-group to a heparin molecule.
Figure 3 is a reaction scheme of grafting heparin molecules to the side chains of poly(valerolactone-co-caprolactone) by click chemistry.
Figure 4 is a schematic view showing a biodegradable polyester heparin conjugate of the present invention applied to the surface of an implantable medical device.
Figure 5 is a schematic view showing a biodegradable polyester heparin conjugate of the present invention combined with a drug to form nanoparticles or microspheres.
Figure 6 is an isometric view of an expandable medical device with particles selectively placed into structural features of the device.
Figure 7 is a cross sectional view of an expandable medical device having particles in accordance with the present invention in a first plurality of holes.

As used herein, "stent" means a generally tubular structure constructed from any biocompatible material that is inserted into a conduit to keep the lumen open and prevent closure due to a stricture or external compression.

As used herein, "biologically active agent" means a drug or other substance that has therapeutic value to a living organism including without limitation antithrombotics, anticancer agents, anticoagulants, antiplatelet agents, thrombolytics, antiproliferatives, anti-inflammatories, agents that inhibit restenosis, smooth muscle cell inhibitors, antibiotics, and the like, and/or mixtures thereof and/or any substance that may assist another substance in performing the function of providing therapeutic value to a living organism.

Exemplary anticancer drugs include acivicin, aclarubicin, acodazole, acronycine, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus calmette-guerin (BCG), Baker's Antifol (soluble), beta-2'-deoxythioguanosine, bisantrene hcl, bleomycin sulphate, busulphan, buthionine sulphoximine, ceracemide, carbetimer, carboplatin, carmustine, chlorambucil, chloroquinoxaline-sulphonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HCl, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, edatrexate, edelfosine, eflomithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5-fluorouracil, Fluosol^{®}, flutamide, gallium nitrate, gemcitabine, goserelin acetate, hepsulpham, hexamethylene bisacetamide, homoharringtonine, hydrazine sulphate, 4-hydroxyandrostenedione, hydrozyurea, idarubicin HCl, ifosfamide, interferon alfa, interferon beta, interferon gamma, interleukin-1 alpha and beta, interleukin-3, interleukin-4, interleukin-6, 4-ipomeanol, iproplatin, isotretinoin, leucovorin calcium, leuprolide acetate, levamisole, liposomal daunorubicin, liposome encapsulated doxorubicin, lomustine, lonidamine, maytansine, mechlorethamine hydrochloride, melphalan, menogaril, merbarone, 6-mercaptopurine, mesna, methanol extraction residue of Bacillus calmette-guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, tumor necrosis factor, uracil mustard, vinblastine sulphate, vincristine sulphate, vindesine, vinorelbine, vinzolidine, Yoshi 864, zorubicin, and mixtures thereof.

Exemplary antiinflammatory drugs include classic non-steroidal anti-inflammatory drugs (NSAIDS), such as aspirin, diclofenac, indomethacin, sulindac, ketoprofen, flurbiprofen, ibuprofen, naproxen, piroxicam, tenoxicam, tolmetin, ketorolac, oxaprosin, mefenamic acid, fenoprofen, nambumetone (relafen), acetaminophen (Tylenol^{®}), and mixtures thereof; COX-2 inhibitors, such as nimesulide, NS-398, flosulid, L-745337, celecoxib, rofecoxib, SC-57666, DuP-697, parecoxib sodium, JTE-522, valdecoxib, SC-58125, etoricoxib, RS-57067, L-748780, L-761066, APHS, etodolac, meloxicam, S-2474, and mixtures thereof; glucocorticoids, such as hydrocortisone, cortisone, prednisone, prednisolone, methylprednisolone, meprednisone, triamcinolone, paramethasone, fluprednisolone, betamethasone, dexamethasone, fludrocortisone, desoxycorticosterone, and mixtures thereof; and mixtures thereof.

As used herein, "effective amount" means an amount of pharmacologically active agent that is nontoxic but sufficient to provide the desired local or systemic effect and performance at a reasonable benefit/risk ratio attending any medical treatment.

In accordance with the present invention, one or more layers of polymeric compositions are applied to a medical device to provide a coating thereto or are formed into particles that are loaded within a structural feature of the medical device. When employed as a coating, the polymeric compositions perform differing functions. For example, one layer may comprise a base coat that allows additional layers to adhere thereto. An additional layer(s) can carry bioactive agents within their polymer matrices. Alternatively, a single coat may be applied wherein the polymeric composition is such that the coat performs multiple functions, such as allowing the coating to adhere to the device and housing an agent that prevents thrombosis. Other functions include housing an agent to prevent restenosis.

The chemical nature of an agent can limit the number of agents that a coating may carry. For example, an antithrombotic agent tends to be hydrophilic while an antiproliferative agent tends to be comparatively hydrophobic. Hence, it is desired to entrap a hydrophobic agent within the matrix of a polymer coating to limit its exposure to water and control its elution from the matrix. The present invention supports two agents having differing properties in close proximity by providing a conjugate between an anti-coagulant such as heparin and a bioabsorbable polymer with a free carboxyl end group. This configuration will result in the hydrophilic heparin agent being oriented substantially away from the hydrophobic agent that resides within the polymer matrix. Thus, when applied to a medical device the coating having the conjugate ensures that the anti-thrombotic agent is substantially oriented away from any hydrophobic agents that may be contained within the polymer matrix.

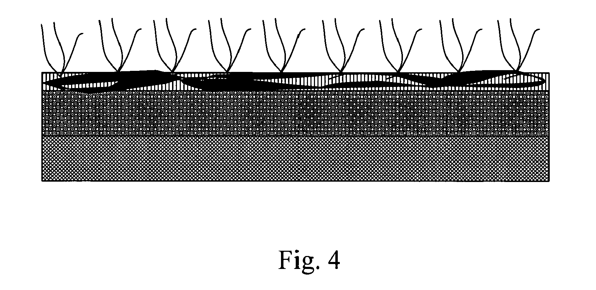

Fig. 4 illustrates an exemplary embodiment of a coating(s) of the present invention applied a surface 2. The surface 2 is located on, for example, an implantable medical device. The coating comprises a first or inner layer 4 of polymeric film loaded with a biologically active agent that, for example, prevents smooth cell proliferation and migration. First layer or coating 4 may contain more than one biologically active agent.

One manner in which the agent is placed within the matrix of the polymer involves using a solvent or mixture of solvents whereby the agent and polymer are dissolved therein. As the mixture dries, the solvent is removed leaving the agent entrapped within the matrix of the polymer. Exemplary polymers that can be used for making the inner/ first polymeric layer include polyurethanes, polyethylene terephthalate (PET), PLLA-polyglycolic acid (PGA) copolymer (PLGA), polycaprolactone (PCL) poly-(hydroxybutyrate/hydroxyvalerate) copolymer (PHBV), poly(vinylpyrrolidone) (PVP), polytetrafluoroethylene (PTFE, such as that sold under the trade mark Teflon), poly(2-hydroxyethylmethacrylate) (poly-HEMA), poly(ether urethane urea), silicones, acrylics, epoxides, polyesters, urethanes, parlenes, polyphosphazene polymers, fluoropolymers, polyamides, polyolefins, and mixtures thereof. Exemplary bioabsorbable polymers that can be used for making the inner/ first polymeric film include polycaprolactone (PCL), poly-D, L-lactic acid (DL-PLA), poly-L-lactic acid (L-PLA), poly(hydroxybutyrate), polydioxanone, polyorthoester, polyanhydride, poly(glycolic acid), polyphosphoester, poly (amino acids), poly(trimethylene carbonate), poly(iminocarbonate), polyalkylene oxalates, polyphosphazenes, and aliphatic polycarbonates.

A second or outmost layer 6 may comprise an anti-thrombotic heparin-bioabsorbable polymer conjugate with strong anticoagulation properties. The second layer of anti-thrombotic heparin-bioabsorbable polymer conjugate may additionally have the effect of preventing a burst release of the biologically active agent dispersed in the first or inner 4 layer, resulting in a relatively longer release period of the layer 4 may contain more than one biologically active agent. In addition, the conjugate 6 orients the hydrophilic heparin 8 substantially away from the hydrophobic inner layer 4.

For purposes of illustrating the present invention only, the coating(s) are discussed as being applied to a medical device such as stents. The coating of the present invention may be employed in other applications such as on a vascular graft, a wound patch, a closure device, a shunt or any other device, covering or the like where it is desired to stop the formation of thrombosis and/or deliver an agent targeted to treat the area of application. In general, stents are made from metal such as those manufactured from stainless steel or cobalt chromium alloys. Stents may, however, also be manufactured from polymeric materials. It is also to be understood that any substrate, medical device, or part thereof having contact with organic fluid, or the like, may also be coated with the present invention. For example, other devices such as vena cava filters and anastomosis devices may be used with coatings having agents therein or the devices themselves may be fabricated with polymeric materials that have the drugs contained therein. Any of the stents or other medical devices described herein may be utilized for local or regional drug delivery. Balloon expandable stents may be utilized in any number of vessels or conduits, and are particularly well suited for use in coronary arteries. Self-expanding stents, on the other hand, are particularly well suited for use in vessels where crush recovery is a critical factor, for example, in the carotid artery.

It is desirable, but not required, that the first 4 and second 6 coatings or layers cover at least a portion of the entire stent surface 2. The application of the first layer 4 is accomplished through a solvent evaporation process or some other known method such as solvent cast spray coating. The solvent evaporation process entails combining the polymeric material and the biologically active agent with a solvent, such as tetrahydrofuran (THF), which are then stirred to form a mixture. An illustrative polymeric material of the first layer comprises polyurethane and an illustrative biologically active agent comprises a rapamycin. The mixture is applied to the surface 2 of the stent by either spraying the solution onto the stent; or dipping the stent into the solution. After the mixture has been applied, the stent is subjected to a drying process, during which, the solvent evaporates and the polymeric material and biologically active agent form a thin film on the stent. Alternatively, a plurality of biologically active agents can be added to the first layer 4.

The second or outmost layer 6 of the stent coating comprises an anti-thrombotic heparin-bioabsorbable polymer conjugate. The anti-thrombotic heparin-bioabsorbable polymer conjugate may be soluble in organic solvents or mixtures of organic solvents of varying polarity. The heparin 8 may comprise an unfracationated heparain, fractionated heparin, a low molecular weight heparin, a desulphated heparin and heparins of various mammalian sources. Exemplary anti-thrombotic agents may include: Vitamin K antagonist such as Acenocoumarol, Clorindione, Dicumarol (Dicoumarol), Diphenadione, Ethyl biscoumacetate, Phenprocoumon, Phenindione, Tioclomarol, Warfarin; Heparin group anti-platelet aggregation inhibitors such as Antithrombin III, Bemiparin, Dalteparin, Danaparoid, Enoxaparin, Heparin, Nadroparin, Parnaparin, Reviparin, Sulodexide, Tinzaparin; other platelet aggregation inhibitors such as Abciximab, Acetylsalicylic acid (Aspirin), Aloxiprin, Beraprost, Ditazole, Carbasalate calcium, Cloricromen, Clopidogrel, Dipyridamole, Eptifibatide, Indobufen, Iloprost, Picotamide, Prasugrel, Prostacyclin, Ticlopidine, Tirofiban, Treprostinil, Triflusal; enzymatic anticoagulants such as Alteplase, Ancrod, Anistreplase, Brinase, Drotrecogin alfa, Fibrinolysin, Protein C, Reteplase, Saruplase, Streptokinase, Tenecteplase, Urokinase; direct thrombin inhibitors such as Argatroban, Bivalirudin, Dabigatran, Desirudin, Hirudin, Lepirudin, Melagatran, Ximelagatran; and other antithrombotics such as Dabigatran, Defibrotide, Dermatan sulphate, Fondaparinux, Rivaroxaban.

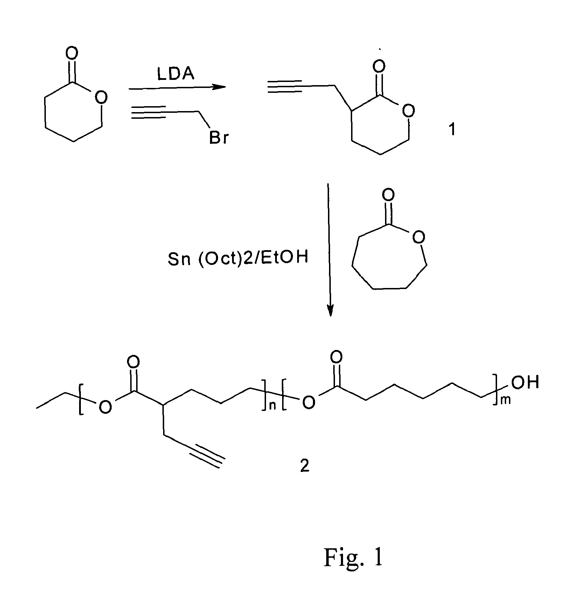

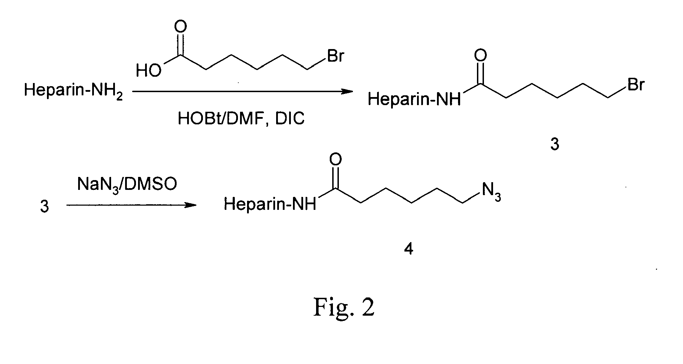

As shown in Figures 1 and 2, an exemplary anti-thrombotic heparin-biocompatible copolymer conjugate is prepared as follows. First, as shown in Figure 1, an acetylene group is introduced to the alpha position of a cyclic monomer valeroclatone, which is copolymerized with caprolactone in the presence of a tin catalyst Sn(OTf)₂ and a predetermined amount of ethanol as the ring opening initiator. The ring opening polymerization results in a copolymer polyester of poly(valerolactone-co-caprolacone) with pendant acetylene groups. The density of the pendant acetylene groups along the polymer chain is determined by the ratio between the functionalized valerolactone and caprolactone used in the polymerization process. The molecular weight of the copolymer is determined by the ratio between the sum of the two monomers and the initiator ethanol. The higher the ratio between the sum of the two monomers and the initiator ethanol, the higher the molecular weight of the final copolymer.

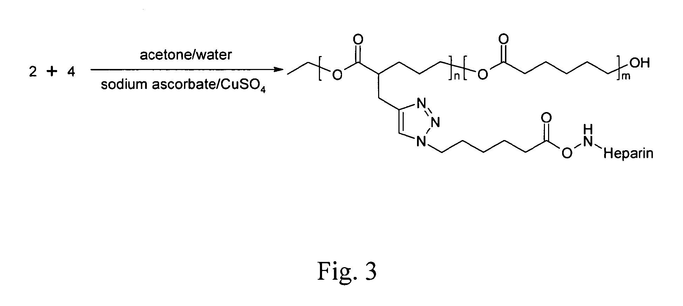

In one embodiment of the present invention the pendant acetylene groups of the copolymer is further reacted with a heparin molecule having azide groups prepared according to the scheme shown in figure 2. The reaction between the acetylene group and azide group in the presence of Cu(I) catalyst to form a triazole linkage is shown in figure 3. Although any heparin molecule, a recombinant heparin, heparin derivatives or heparin analogues (having a preferred weight of 1,000 to 1,000,000 daltons) may be used in the coupling reaction to make the final anti-thrombotic heparin-bioabsorbable polymer conjugate, it is preferred to use a desulphated heparin to increase the coupling efficiency of the reaction.

Once the anti-thrombotic heparin-bioabsorbable polymer conjugate is prepared, it may be applied directly over a first layer using the solvent evaporation method or other appropriate method. After the solvent is evaporated from the surface of an implantable medical device, a thin film comprising the anti-thrombotic heparin-bioabsorbable polymer conjugate remains on the outmost surface of the device.

The following examples illustrate the creation of the conjugate and uses in accordance with the principle of the present invention.

### Example 1: Preparation Of an acetylene-containing valerolactone monomer

As shown in Figure 1, a pre-determined amount of delta-valerolactone (technical grade from Aldrich, USA) is reacted with N,N-diisopropylamide (LDA, 99.5+%, Aldrich, USA) in tetrahydrofuran (THF) at -78° C, followed by quenching with a toluene solution of propargyl bromide (80 wt% toluene solution, Aldrich, USA). Kugelrohr distillation of the crude product at 140° C gave lactone 1 as a colorless, viscous liquid.

### Example 2: Copolymerization of valerolactone having an acetylene side chain with caprolactone via a ring-opening polymerization with ethanol as the initiator

Purified valerolacone monomer made in example I is copolymerized with epsilon-caprolactone (99+%, Aldrich, USA) in a dried round bottom glass reactor equipped with a magnetic stir bar, with ethanol (anhydrous grade, Aldrich, USA) as the initiator and Sn(OTf)₂ as the ring-opening catalyst. The reaction scheme is shown in figure 1. The ring-opening polymerization is performed neat or using toluene as solvent at room temperature or at an elevated temperature. The molecular weight of the copolymers increase with time of reaction and the use of Sn(OTf)₂ offers a better control of polydispersity of the final copolymer compared with the more conventional Tin-based catalyst such as stannous Octoate. The density of the pendant acetylene group in the block polyester copolymer is adjusted by varying the molar ratio between the valerolactone monomer and epsilon-caprolactone (CL)

Similarly other lactone monomers such as lactide (LA) and glycolide (GA) may be used in the copolymerization to adjust the physical properties of the functionalized polyester copolymers. The final percentage of incorporation of acetylene group may be calculated based on the ratio of H1-NMR spectral signals at 2.01 (acetylene proton from monomer 1) against the signal at 4.03 (CH₂O of the polymer backbone from monomer 1 and CL).

### Example 3: Introduction of an azide end-group to a heparin molecule

Azide terminated heparin is synthesized via a route as shown in figure 2. Briefly, a solution of bromohexanoic acid and 1-hydroxybenzotriazole (HOBt, < 5% water, Aldrich, USA) are added to dry DMF. N, N'-diisoporprylcarbodiimide (DIC, 99%, Aldrich, USA) is added dropwise to the solution and stirred for 20 min. The activated solution is added to a heparin solution in DMF and agitated for 1 hour. The reaction is then filtered to remove solids. The crude product is then concentration by rotary evaporation and precipitated in ether. The precipitate is then washed 3 times with ether and vacuum dried overnight. The bromide-terminated heparin is then dissolved in DMSO and sodium azide is added to the solution. The reaction is allowed to proceed for 12 hours at room temperature after which the solution is filtered. Following rotary evaporation and Kugelrohr distillation to remove DMSO, the crude product is dissolved in a minimal amount of methanol and the insoluble precipitate is removed by filtration. The remaining solution is precipitated from diethyl ether and filtered to yield azide-terminated heparin.

### Example 4: Grafting of azide-terminated heparin to acetylene side chains of polyester copolymer by click chemistry

The grafting of azide-capped heparin molecules to the acetylene side chains of the polyester copolymers by click chemistry is carried out in the following conditions. Heparin-azide is first dissolved in water in a reaction vessel. Polyester copolymer with acetylene side chains is dissolved in minimal amount of acetone and added by syringe to the rapidly stirred reaction mixture. Sodium ascorbate and copper (II) sulphate pentahydrate are then added to the reaction vessel. The reaction mixture is then heated to 80°C until bubbling due to evaporation of acetone completes. The vessel is then fitted with a condenser, heated to reflux and stirred overnight. The reaction mixture is then cooled to room temperature, diluted with a saturated NaCl aqueous solution, and extracted 5 times with CH₂Cl₂. The combined organic layers are then dried over MgSO₄ and concentrated by rotary evaporation. The resulting product is then dried overnight under vacuum to yield the final polymer heparin conjugate.

### Example 5: Coating Of A Drug Eluting Stent With An Outmost Layer Comprising An Absorbable polyester-Heparin Conjugate

As shown in Figure 4, the surface 10 of a cobalt chromium stent is spray coated with a drug containing polymeric solution, which may comprise for example, ethyl acetate (EA) containing PLGA and rapamycin. The weight ratio between PLGA and rapamycin is 2:1. After the drug-containing layer 20 is dried, a coating solution containing an absorbable polyester-heparin conjugate of the present invention is spray coated onto the first drug-containing layer 20. After the layer is dried, a thin film 30 containing the absorbable polyester-heparin conjugate is formed on the outmost surface.

Coatings such as those described above can be thin, typically 5 to 8 microns deep. The surface area of a device such as a stent, by comparison is very large, so that the entire volume of the beneficial agent has a very short diffusion path to discharge into the surrounding tissue. The resulting cumulative drug release profile is characterized by a large initial burst, followed by a rapid approach to an asymptote, rather than the desired "uniform, prolonged release," or linear release. It is often desired to vary the elution pattern of a therapeutic agent from a device such as a stent. In addition, it is also desired to vary the amount of agent at different locations along the device. This can be accomplished by placing an agent within a structural feature of the device.

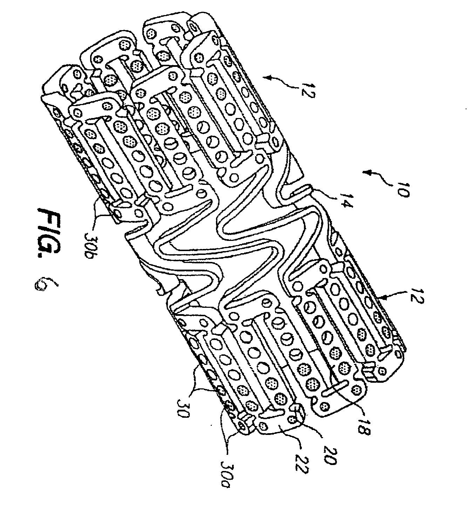

As shown in Figure 6, an expandable device has a plurality of structural features that facilitate the placement of at least one agent on the device. The expandable medical device 10 illustrated in Figure 6 may be cut from a tube of material to form a cylindrical expandable device. The expandable medical device 10 includes a plurality of cylindrical sections 12 interconnected by a plurality of bridging elements 14. The bridging elements 14 allow the device to bend axially when passing through the torturous path of vasculature to a deployment site and allow the device to bend axially when necessary to match the curvature of a lumen. A network of elongated struts 18 that are interconnected by ductile hinges 20 and circumferential struts 22 comprise the cylindrical tubes 12. During expansion of the medical device 10 the ductile hinges 20 deform while the struts 18 are not deformed. Further details of an example of the expandable medical device are described in US-6241,762.

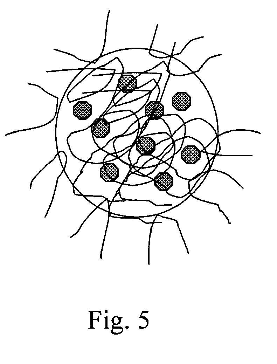

The elongated struts 18 and circumferential struts 22 include structural features such as openings 30, some of which are selectively filled with an agent for delivery to the lumen in which the expandable medical device is implanted. The depth of the openings 30 is dictated by the thickness of the struts 22. Other structural features may include raised sections or dimples, slits, elongated openings, added material and any feature that can capture or contain a material that is desired to be placed on the expandable device. In addition, other portions of the device 10, such as the bridging elements 14, may include structural features. In the particular example shown in Figure 5, the openings 30 are provided in non-deforming portions of the device 10, such as the struts 18, so that the openings are non-deforming and the agent is delivered without risk of being fractured, expelled, or otherwise damaged during expansion of the device. A further description of one example of the manner in which the beneficial agent may be loaded within the openings 30 is described in US-6764507.

In order to facilitate the placement of an agent or multiple agents within a structural feature of a device as shown in Figure 5, a particle 40 can be created utilizing the polymer and heparin conjugate as a carrier for the therapeutic agent as shown in Figure 4. In this embodiment the agent 42 is somewhat associated with the hydrophobic core 46 of the comb polymer 44. The agent 42 is co-dissolved with the conjugate using a solvent that is later evaporated creating particles with the agent at their core. These particles are ideally suited for placement within the structure of a device such as illustrated in Figure 6. For example, a device may have structural features such as wells, indentations, folds, or channels having particles therein. This allows for particles having differing properties to be placed at various locations along the device. Moreover, particles having at least two different agents can be located within the same structural feature. Agent is released from the structural feature as the particles degrade. Simultaneously, the presence of heparin will prevent thrombosis at the placement site of the device.

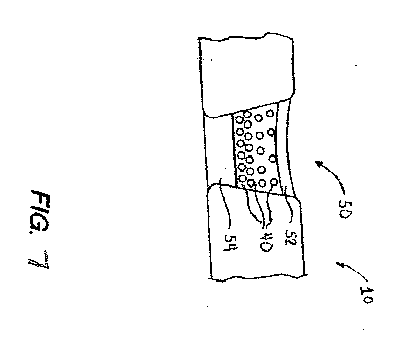

Figure 7 illustrates a cross sectional view of an opening 50 in the device 10 of Figure 5. A plurality of particles 40 is placed between two layers 52 and 54. Layers 52 and 54 can be varied in composition and thickness to control the exposure of particles 40 to an aqueous environment. This will control the release of agent from within the core of the particles 40. Additionally, the particles can be blended within a single material and placed within opening 50 of device 10.

Examples of methods for the formation of nanoparticles and microparticles for placement on or within a structural feature of a device are described below.

### Example 6: Formation of nanoparticles using a comb-type absorbable polymer-heparin and paclitaxel

Twenty mg of Paclitaxel and 200 mg of poly(lactide-to-glycolide), PLGA50/50, are dissolved 16 ml of methylene chloride with gentle stirring. The formed solution is transferred to 250 ml of aqueous solution containing 4 % of (polyvinyl alcohol) (PVA) as an emulsifier. The combined solution is sonicated with an energy output of 50 mW in a pulsed mode of a sonicator for 90 seconds. The emulsion is then stirred overnight at room temperature to remove the solvent. This forms nanospheres containing paclitaxel that are collected by centrifugation at 12000 rpm for 30 min and further washed with deionized water 4 times to remove excess emulsifiers. The product is then freeze-dried before application.

### Example 7: Formation of microparticles using a comb-type absorbable polymer-heparin and paclitaxel

Twenty mg of Paclitaxel and 200 mg of poly(lactide-to-glycolide), PLGA50/50, are dissolved 16 ml of ethyl acetate (EA) with gentle stirring. Eighty ml of water (water for injection grade) is heated up to 50 C and kept stirred by a magnetic stirring plate. A predetermined amount of emulsifier (PVA, 0.4 g) is added to form an aqueous solution. The solution is then cooled to room temperature under constant stirring. Ethyl acetate (3.2 ml) is added to the aqueous solution under gentle stirring. Paclitaxel and PLGA solution is then slowly poured to the emulsified aqueous solution that is being stirred at 500 rpm. The emulsion is further stirred for 4 hours at room temperature to solidify the microspheres. The final microspheres are then collected by filtration and washed 2 times with WFI water. The final microspheres are freeze-dried over night before subsequent use.

Figure 5 shows particles made in accordance with the above-examples placed within an opening of the device shown in Figure 6. The particles may be placed within these openings by a dry powder deposition method such as an electrostatic deposition process. These particle containing device may be further process to modulate the release kinetics of the drug with a process such as a solvent spray process to further modulate the release kinetics the opening may also be covered by additional coverings to adjust the release kinetics of the drug.

## Claims

1. A method for forming an anti-thrombotic conjugate comprising the steps of:
providing at least one cyclic lactone molecule; and
introducing an alkyne group to an alpha position of a carbonyl group of the cyclic lactone molecule;
polymerizing the cyclic lactone in a ring opening polymerization;
derivatizing an antithrombotic agent with an azide end group; and
forming an antithrombotic conjugate via a triazole linkage by a click chemistry process between the alkyne group and the azide group.

2. The method of claim 1 wherein the at least one cyclic lactone molecule comprises a glycolide.

3. The method of claim 1 wherein the at least one cyclic lactone molecule comprises caprolactone.

4. The method of claim 1 further comprising the step of providing a second lactone molecule.

5. The method of claim 1 wherein click chemistry process is accomplished via a triazole linkage between the side chain of the polyester and the heparin.

6. A conjugate material comprising a biocompatible and bioabsorbable polymer and an antithrombotic agent attached as side chains via a click chemistry process.

7. The conjugate material of claim 6, in which the antithrombotic agent is attached to the polymer through triazole linkages resulting from a reaction between an azide end group on a precursor to the antithrombotic agent and an alkyne group.

8. The conjugate material of claim 6, wherein the anti-thrombotic agent is a heparin, preferably a de-sulphated heparin.

9. The conjugate material of claim 6, wherein the bioabsorbable polyester is selected from a group consisting of polycaprolactone (PCL), poly-D, L-lactic acid (DL-PLA), poly-L-lactic acid (L-PLA), poly(glycolic acid) (PGA), poly(lactic acid -co-glycolic acid) (PLGA), poly(hydroxybutyrate), poly(hydroxybutyrate-co-valerate), polydioxanone, poly(glycolic acid-co-trimethylene carbonate).

10. The conjugate material of claim 6, wherein the biocompatible polymer comprises polyvalerolactone/polycaprolactone copolymer and the anti-thrombotic agent comprises a heparin molecule, the conjugate having the following structure: 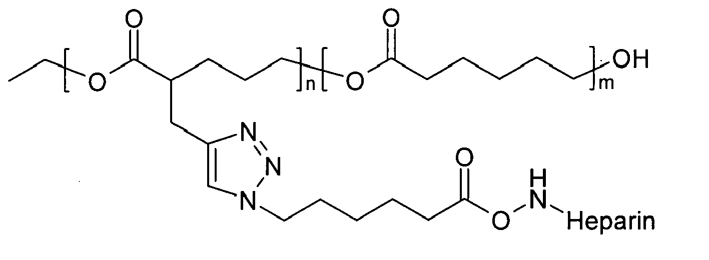 wherein n and m are each an integer of 2 to 5000.

11. A coated article comprising:
a first bioabsorbable polymer applied to a surface of the article;
an agent contained within the first bioabsorbable polymer; and
a conjugate material comprising a biocompatible and bioabsorbable polymer and an antithrombotic agent attached as side chains via a click chemistry process wherein the conjugate material is applied to the top of the first bioabsorbable polymer.

12. The article of claim 11, wherein the anti-thrombotic molecule comprises at least one of a heparin, rapamycin, paclitaxel and pimecrolimus.

13. The article of claim 11, wherein the first bioabsorbable polymer comprises a first copolymer, and second bioabsorbable polymer comprises a bioabsorbable polymer wherein at least one antithrombotic agent was conjugated to the polymer backbone via a triazole linkage by a click chemistry process.

14. The conjugate material of claim 13, wherein the first and second copolymers are the same and are selected from a group consisting of polycaprolactone (PCL), poly-D, L-lactic acid (DL-PLA), poly-L-lactic acid (L-PLA), poly(glycolic acid) (PGA), poly(lactic acid -co-glycolic acid) (PLGA), poly(hydroxybutyrate), polyvalerate poly(hydroxybutyrate-co-valerate), polydioxanone, poly(glycolic acid-co-trimethylene carbonate).

15. A method of making a plurality of particles comprising the steps of:
forming a conjugate between a biocompatible and bioabsorbable polymer and an anti-thrombotic agent via a click chemistry process;
dissolving a therapeutic agent and a polymeric anti-thrombotic agent conjugate in at least one solvent to form a first solution;
mixing the first solution with a second aqueous solution comprising water and at least one surfactant to form an emulsion; and
removing the solvent from the emulsion to form a plurality of particles.

16. The method of claim 15, wherein the biocompatible and bioabsorbable copolymer comprises a terpolymer of valerolactone, d,1-polylactide, and at least one of glycolide and caprolactone, and the anti-thrombotic agent comprises a heparin molecule.

17. An apparatus comprising:
a frame expandable from a first diameter to a second diameter wherein the frame has an inner surface and an outer surface, the distance between the surfaces defining the wall thickness of the frame;
a plurality of structural features disposed along the frame; and
a plurality of bioabsorbable polymer anti-thrombotic conjugate particles situated with the plurality of structural features, which preferably serve as a carrier for a therapeutic agent.

18. The apparatus of claim 17, wherein the plurality of structural features comprise (a) ridges disposed on the surface of the frame, or (b) a plurality of wells formed in the frame, in which the wells preferably extend from the outer surface to the inner surface.
